**Europäisches Patentamt**

**European Patent Office**

**Office européen des brevets**

(19)

(11) Publication number : **0 325 244 B1**

(12)

# EUROPEAN PATENT SPECIFICATION

(45) Date of publication of patent specification :
08.04.92 Bulletin 92/15

(51) Int. Cl.⁵ : **A61K 47/00**

(21) Application number : **89100869.0**

(22) Date of filing : **19.01.89**

(54) **Aqueous solution of fat-soluble substance.**

(30) Priority : **22.01.88 JP 12063/88**

(43) Date of publication of application :
**26.07.89 Bulletin 89/30**

(45) Publication of the grant of the patent :
**08.04.92 Bulletin 92/15**

(84) Designated Contracting States :
**AT BE CH DE ES FR GB IT LI NL SE**

(56) References cited :
**EP-A- 0 132 821**
**EP-A- 0 144 434**
**EP-A- 0 214 501**

(73) Proprietor : **Eisai Co., Ltd.**
**6-10, Koishikawa 4-chome Bunkyo-ku**
**Tokyo 112 (JP)**

(72) Inventor : **Shibusawa, Koichi**
**387-15, Yogemachi**
**Isezaki-shi Gunma (JP)**
Inventor : **Ohsawa, Shigemitsu**
**2286-12, Suehirocho Honjo-shi**
**Saitama (JP)**

(74) Representative : **Hansen, Bernd, Dr.rer.nat. et al**
**Hoffmann, Eitle & Partner Patentanwälte**
**Arabellastrasse 4 Postfach 81 04 20**
**W-8000 München 81 (DE)**

## Description

The invention relates to an aqueous solution of a fat-soluble substance, for example, a fat-soluble vitamin, and provides a solution, uniformly emulsified or solubilized, which is obtained even with mild stirring.

In the state of the art, an aqueous solution of a fat-soluble substance, a phosphatide and a polyhydric alcohol is obtained with the use of a high pressure homogenizer, a ultrasonic emulsifying machine for a long time such as 60 minutes. This strong dispersing power is indeed necessary which is why mass-production cannot be conducted.

In the invention, instead of such a machine, a device effecting mild stirring, for example Polytron® and a homomixer, is available to offer a mild share stress. Alternatively an ultra-sonic emulsifying machine can be used for a short time. This can be advantageously attained with incorporation of a basic amino acid, a salt thereof or Meglumin into the solution. The invention provides an aqueous solution which comprises a fat-soluble substance, a phosphatide, a polyhydric alcohol and a basic amino acid, a salt thereof or Meglumine.

Thus, the invention provides an aqueous solution comprising:

(a) a fat-soluble substance selected from vitamin E acetate, coenzyme Q10, vitamin A, an aliphatic ester of vitamin C, emulsified or solubilized in the aqueous phase;

(b) a phosphatide selected from soy bean lecithin and egg yolk lecithin;

(c) a polyhydric alcohol selected from glycerin and D-sorbitol;

d) 0.1 to 0.5 parts by weight, per 1 part of (b) of a basic amino acid selected from L-arginine, L-lysine or a salt thereof or Meglumine (N-methylglucamine); and

(e) water.

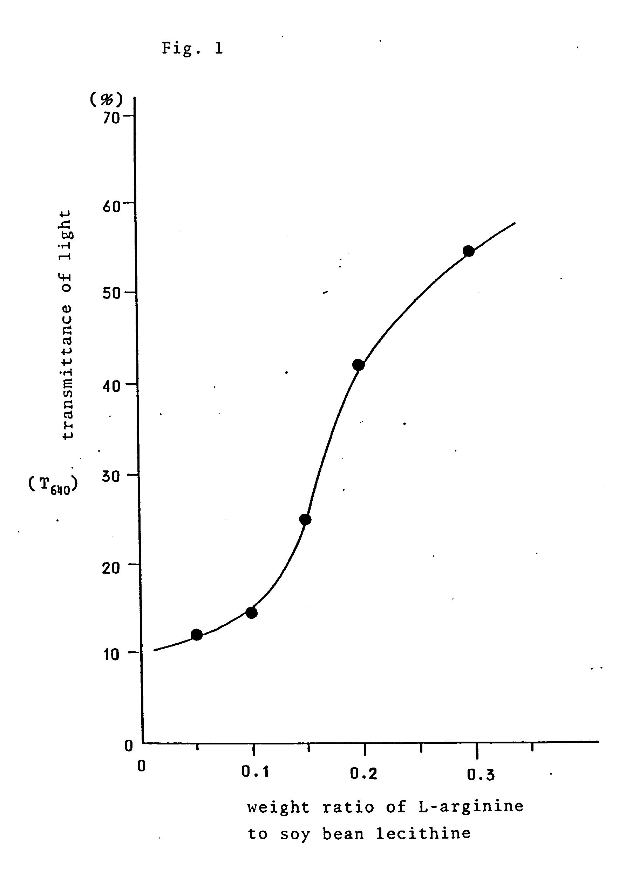

Fig. 1 shows results of Test III.

### Example 1

An aqueous solution was prepared in the following way. Solution A comprising 100 mg of vitamin E acetate, 30 mg of L-arginine, 100 mg of purified soy bean lecithin and 770 mg of glycerine was prepared with a Polytron®. 19 of Solution A was mixed with 12 ml of purified water, while stirred. Mixture B comprising 0.5 ml of ginseng extract liquid, 25 mg of nicotinamide, 10 mg of FMN(VB2), 50 mg of caffein, 3000 mg of D-sorbitol, 5000 mg of purified white sugar, 18 mg of benzoic acid, 20 mg of dl-malic acid and a suitable amount of sodium citrate was dissolved in 12 ml of purified water. Mixture D comprising 3 mg of ethyl paraben, 1 ml of ethanol and a trace of a perfume was added to and mixed with the Solution B. Solutions A and B were mixed together and the mixture was adjusted with sodium citrate to a pH of 5.5. More purified water was added to a total volume of 30 ml. The final liquid was found to be transparent and clear.

### Example 2

Mixture B comprising 0.3 g of Meglumin, 6.9 g of glycerine and 0.8 ml of ethanol was heated to obtain a solution. Mixture A comprising 1.0 g of soy bean oil and 1.0 g of soy bean phosphatide was added to Solution B. A clear solution was obtained with the use of a Polytron®.

### Example 3

3 mg of Meglumin was dissolved in a mixture of 27 mg of glycerine and 50 mg of a 70% solution of D-sorbitol. 10 mg of coenzyme Q and 10 mg of purified egg yolk lecithine were added to the solution. The mixture was treated with a Polytron® to an aqueous solution. The solution was further diluted with water and adusted with a suitable amount of citric acid solution in purified water for injection to a pH of about 7 and obtain 1 ml of a clear injectable liquid.

### Test I

The aqueous solutions listed in Table 1 were prepared and examined for water dispersibility. The components 1 and 4 were dissolved in water, while heated. Then components 2 and 3 were added to the solution. The mixture was treated with a Polytron® at 7000 RPM for 30 minutes. The obtained solutions were observed in respect to their appearance. A 3 % dispersion of each solution in water was obtained and it was observed. Moreover it was determined in view of transmittance of light at O.D. 640 nm.

Table 1

| | composition | | |
|---|---|---|---|
| | 1 | 2 | 3 |
| **component** | | | |
| 1  L-arginine | – | 0.3 g | – |
|    Meglumin | – | – | 0.3 g |
| 2  soy bean lecithine | 1.0 g | 1.0 g | 1.0 g |
| 3  vitamin E acetate | 1.0 g | 1.0 g | 1.0 g |
| 4  glycerine | 8.0 g | 7.7 g | 7.7 g |
| appearance | milky | clear | clear |
| dispersibility in water | milky | clear | clear |
| transmittance at 640 nm | separates | over 30% | over 20% |

The composition 1 is control and the compositions 2 and 3 fall within the invention. The composition 1 was found to have oil and lecithin drops floating on the surface.

Test II

Aqueous solutions were prepared, as listed in Table 2, with different of amounts of L-arginine. They were tested for water dispersibility. The results are shown in Table 2. L-arginine was dissolved in glycerine with heating. Then, soy bean lecithin and vitamin E acetate were added to the solution and the mixture was treated with a Polytron® at 7000 RPM for 30 minutes. They were observed in appearance. A 3% aqueous solution of each resultant was determined in view of transmittance of light at O.D. 640 nm.

Table 2

| composition | 0/10 | 0.5/10 | 1/10 | 2/10 | 3/10 | 5/10 |
|---|---|---|---|---|---|---|
| L-arginine | 0 | 0.05 | 0.1 | 0.2 | 0.3 | 0.5 |
| soy bean lecithine | 1 | 1 | 1 | 1 | 1 | 1 |
| vitamin E acetate | 1 | 1 | 1 | 1 | 1 | 1 |
| glycerine | 8 | 7.95 | 7.9 | 7.8 | 7.7 | 7.5 |
| appearance | milky | clear | clear | clear | clear | clear |
| tansmittance at 640 nm (%) | – | 10 | 15 | 20 | 30 | 40 |

Ratios in the uppermost column show those of L-arginine to lecithine. In the test on 0/10 in transmittace, some oil was found to separate out.

Test III

Aqueous solutions were prepared containing different ratios of L-arginine to soy bean lecithin. The L-arginine was dissolved, while heated, in glycerin. Soy bean lecithin and vitamin E acetate were then added to the solution and the mixture was stirred with an ultra-sonic emulsifying machine for 90 seconds. The thus obtained aqueous liquids were adjusted to have a concentration of 3 % by weight. They were tested in view of light transparency at O.D. 640 nm. The results given in Fig. 1 show that addition of L-arginine was found to be advantageous in comparison with no addition.

## Claims

**Claims for the following Contractings States : AT, BE, CH, DE, FR, GB, IT, NL, SE**

1. An aqueous solution comprising:
(a) a fat-soluble substance selected from vitamin E acetate, coenzyme Q10, vitamin A, an aliphatic ester of vitamin C, emulsified or solubilized in the aqueous phase;
(b) a phosphatide selected from soy bean lecithin and egg yolk lecithin;
(c) a polyhydric alcohol selected from glycerin and D-sorbitol;
(d) 0.1 to 0.5 parts by weight, per 1 part of (b) of a basic amino acid selected from L-arginine, L-lysine or a salt thereof or Meglumine (N-methylglucamine); and
(e) water.
2. The use of an aqueous solution according to Claim 1 for preparing an injection liquid, an emulsion or an oral preparation.

**Claim for the following Contracting State : ES**

1. A method for preparing an injection liquid, an emulsion or an oral preparation, characterized in that an aqueous solution is used comprising

(a) a fat-soluble substance selected from vitamin E acetate, coenzyme Q10, vitamin A, an aliphatic ester of vitamin C, emulsified or solubilized in the aqueous phase;
(b) a phosphatide selected from soy bean lecithin and egg yolk lecithin;
(c) a polyhydric alcohol selected from glycerin and D-sorbitol;
(d) 0.1 to 0.5 parts by weight, per 1 part of (b) of a basic amino acid selected from L-arginine, L-lysine or a salt thereof or Meglumine (N-methylglucamine); and
(e) water.

## Patentansprüche

### Patentansprüche für folgende Vertragsstaaten : AT, BE, CH, DE, FR, GB, IT, LI, NL, SE

1. Wässrige Lösung, enthaltend:
(a) eine fettlösliche Substanz, ausgewählt aus Vitamin E-Acetat, Coenzym Q10, Vitamin A, einen aliphatischen Ester von Vitamin C, welcher in der wässrigen Phase emulgiert oder solubilisiert ist;
(b) ein Phosphatid, ausgewählt aus Sojabohnenlecitin und Eierdot- terlecitin,
(c) einen mehrwertigen Alkohol, ausgewählt aus Glycerin und D-Sorbitol;
(d) 0,1 bis 0,5 Gew.-Teile auf einen Teil von (b) einer basischen Aminosäure, ausgewählt aus L-Arginin, L-Lysin oder einem Salz davon oder Meglumin (N-Methylglucamin); und
(e) Wasser.
2. Verwendung einer wässrigen Lösung nach Anspruch 1 zur Herstellung einer Injektionsflüssigkeit, einer Emulsion oder einer oralen Präparation.

### Patentanspruch für folgenden Vertragsstaat : ES

1. Verfahren zur Herstellung einer Injektionsflüssigkeit, einer Emulsion oder einer oralen Präparation, dadurch **gekennzeichnet**, daß eine wässrige Lösung verwendet wird, die enthält:
(a) eine fettlösliche Substanz, ausgewählt aus Vitamin E-Acetat, Coenzym Q10, Vitamin A, einen aliphatischen Ester von Vitamin C, welcher emulgiert oder solubilisiert in der wässrigen Phase ist;
(b) ein Phosphatid, ausgewählt aus Sojabohnenlecitin und Eierdot- terlecitin,
(c) einen mehrwertigen Alkohol, ausgewählt aus Glycerin und D-Sorbitol;
(d) 0,1 bis 0,5 Gew.-Teile auf einen Teil von (b) einer basischen Aminosäure, ausgewählt aus L-Arginin, L-Lysin oder einem Salz davon oder Meglumin (N-Methylglucamin); und
(e) Wasser.

## Revendications

### Revendications pour les Etats contractants suivants : AT, BE, CH, DE, FR, GB, IT, LI, NL, SE

1. solution aqueuse comprenant :
(a) une substance soluble dans les graisses choisie parmi l'acétate de vitamine E, le coenzyme Q10, la vitamine A, un ester aliphatique de vitamine C, émulsifiée ou solubilisée dans une phase aqueuse;
(b) un phosphatide choisi entre la lécithine de soja et la lécithine de jaune d'oeuf;
(c) un polyalcool choisi entre la glycérine et le D-sorbitol;
(d) 0,1 à 0,5 parties en poids, pour 1 partie de (b) d'un acide aminé basique choisi parmi la L-arginine, la L-lysine ou un sel de celles-ci ou de Méglumine (N-méthylglucamine); et
(e) de l'eau
2. Utilisation d'une solution aqueuse selon la revendication 1 pour la préparation d'un liquide pour injection, une émulsion ou une préparation orale.

### Revendication pour l'Etat contractant suivant : ES

1. Procédé de préparation d'un liquide injectable, d'une émulsion ou d'une préparation orale, caractérisée en ce qu'on utilise une solution aqueuse comprenant :
(a) une substance soluble dans les graisses choisie parmi l'acétate de vitamine E, le coenzyme Q10, la vitamine A, un ester aliphatique de vitamine C, émulsifiée ou solubilisée dans la phase aqueuse;

(b) un phosphatide choisi entre la lécithine de soja et la lécithine de jaune d'oeuf;

(c) un polyalcool choisi entre la glycérine et le D-sorbitol;

(d) 0,1 à 0,5 parties en poids, pour 1 partie de (b) d'un acide aminé basique choisi parmi la L-arginine, la L-lysine ou un sel de celles-ci ou de Méglumine (N-méthylglucamine); et

(e) de l'eau.

Fig. 1

transmittance of light

$(T_{640})$

(%)

weight ratio of L-arginine
to soy bean lecithine